# EUROPÄISCHE PATENTANMELDUNG

(11) **EP 1 518 865 A1**
(43) Veröffentlichungstag der Anmeldung: **30.03.2005**
(21) Anmeldenummer: 04104520.4
(22) Anmeldetag: 17.09.2004
(51) Int. Cl.: C08B 31/00

(54) **Vernetzte, langzeitstabile Carboxymethylstärke, deren Verwendung als Absorptionsmittel für Wasser sowie Verfahren zu deren Herstellung**

(30) Priorität: 19.09.2003 DE 10343413
(71) Anmelder: Universität Osnabrück, 49069 Osnabrück (DE); Universität Hamburg, 20146 Hamburg (DE)
(72) Erfinder: Lechner, Manfred Dieter, 49134 Wallenhorst (DE); Kulicke, Werner-Michael, 22523 Hamburg (DE); Hess, Christoph, 47533 Kleve (DE); Seidel, Christian, 20251 Hamburg (DE); Hartmann, Brigitte, 49086 Osnabrück (DE)
(74) Vertreter: Eisenführ, Speiser & Partner

(57) **Zusammenfassung**

Beschrieben wird eine vernetzte Carboxymethylstärke zur Verwendung als Absorptionsmittel für Wasser, wobei die Vernetzung teilweise oder vollständig über Dietherbrücken des Typs -O-CH(COO⁻)-O- oder -O-CH(COOH)-O- oder -O-C(CH₃)(COO⁻)-O- oder -O-C(CH₃)(COOH)-O- oder -O-CH(COCH₃)-O- erfolgt, die zwischen benachbarten Stärke-Polymerketten ausgebildet sind.

## Beschreibung

Die vorliegende Erfindung betrifft primär eine neue vemetzte Carboxymethylstärke, die als Absorptionsmittel für Wasser und wässrige Lösungen eingesetzt werden kann. Weitere Aspekte der vorliegenden Erfindung betreffen Hydrogele und sonstige Produkte, die eine erfindungsgemäße vemetzte Carboxymethylstärke umfassen sowie Verfahren zur Herstellung der vemetzten Carboxymethylstärke. Schließlich betrifft die Erfindung auch die Verwendung einer erfindungsgemäßen vernetzten Carboxymethylstärke als Absorptionsmittel für Wasser oder eine wässrige Lösung.

Es sind bereits Absorptionsmaterialien mit hohem Absorptionsvermögen für wässrige Flüssigkeiten bekannt. Diese Absorptionsmittel werden auch als Superabsorber bezeichnet; sie können große Mengen an Flüssigkeit aufnehmen und dauerhaft festhalten. Für Superabsorber existiert ein vielseitiges Anwendungsspektrum, z. B. können Hydrogele auf Basis von Superabsorbern als Kontaktgel für medizinische Ultraschalluntersuchungen eingesetzt werden, d. h. sie können als Medium zwischen Hautoberfläche und Ultraschallkopf dienen.

In der Praxis werden bisher hauptsächlich Superabsorber auf Basis nicht nachwachsender Rohstoffe eingesetzt, die insbesondere synthetisch aus Erdölprodukten hergestellt werden und somit nicht oder nur schlecht biologisch abbaubar sind (dies betrifft insbesondere die üblichen Superabsorber auf Basis von Polyacrylaten und Polyacrylat-Copolymeren). Beim Einsatz in Hygieneprodukten oder bei medizinischen Anwendungen, bei denen ein direkter Hautkontakt vorgesehen ist, kann es bei Einsatz der in der Praxis bislang üblichen Superabsorber zu Hautreizungen kommen. Hydrogele, die einen Superabsorber mit Acrylatstruktur umfassen, sind als nicht unbedenklich einzustufen, insbesondere seitdem Polyacrylate in den MAK-Listen als krebserzeugende Verbindungen der Kategorie 4 (Substanz mit Wirkungsschwelle) eingeordnet werden.

Aus der DE 196 54 745 C2 sind bereits biologisch abbaubare Absorptionsmittel bekannt, die auf nachwachsenden Rohstoffen basieren. Die in dem besagten Dokument beschriebenen Absorptionsmittel sind erhältlich durch Vernetzung eines ionischen Carboxymethyl- oder Carboxy-Polysaccharid-Derivats in Gegenwart von 10 - 80 Gew.-% (bezogen auf das Polysaccharid-Derivat) Wasser bei 110 - 180°C. Das Polysaccharid-Derivat ist dabei beispielsweise eines auf Stärkebasis. Als Vernetzungsmittel werden Dicarbon-, Tricarbon- oder Oligocarbonsäuren eingesetzt; dementsprechend handelt es sich bei den in der DE 196 54 745 C2 beschriebenen Absorptionsmitteln um Polysaccharid-Derivate, bei denen die Vernetzung über Esterbindungen erfolgt. Wenngleich die in der DE 196 54 745 C2 beschriebenen Absorptionsmittel durchaus in der Praxis einsetzbar sind, weisen sie doch bestimmte Nachteile auf. Insbesondere erweisen sich die aus Stärkederivaten synthetisierten Absorptionsmittel, bei denen die Verknüpfungen des Netzwerks über Esterbrücken erfolgen, als nicht langzeitstabil, wenn sie mit Wasser kontaktiert werden. Vielmehr werden die Esterbrücken mit der Zeit hydrolysiert und somit das Absorptionsmittel zersetzt.

US 6 322 724 B1 offenbart vemetzte Stärkederivate als Absorptionsmittel, wobei die Vernetzung teilweise oder vollständig über Etherbrücken des Typs -O-R-O- erfolgt, wobei R eine ggf. Hydroxy-substituierte aliphatische Gruppe mit 1-10 C-Atomen ist.

EP 670 180 A1 offenbart Absorptionsmittel, die eine z.B. carboxymethylierte Stärke umfasssen, welche mittels eines Vernetzers vernetzt ist, bei dem es sich z.B. um Epichlorhydrin handelt.

EP 852 235 A2 offenbart ebenfalls carboxymethylierte Stärkepolymere, die mittels Epichlorhydrin vernetzt sind.

DE 37 50 719 T2 offenbart über Etherverbindungen vernetzte Carboxyl enthaltende Polysaccharide, wobei die Vernetzung mittels bi- oder polyfunktionellen Epoxiden erfolgt.

Es war die Aufgabe der vorliegenden Erfindung, ein langzeitstabiles Absorptionsmittel für wässrige Flüssigkeiten (Wasser, wässrige Lösungen und wässrige Dispersionen) anzugeben. Vorteilhafterweise sollte das anzugebende Absorptionsmittel dabei auch möglichst viele der nachfolgenden Erfordernisse erfüllen:
1. Das Absorptionsmittel sollte auf nativen Ausgangssubstanzen basieren.
2. Aus dem Absorptionsmittel sollten sich Hydrogele mit einer hohen mechanischen Gelfestigkeit bilden lassen.
3. Das Absorptionsmittel und die daraus herstellbaren Hydrogele sollten weitgehend biologisch abbaubar sein.
4. Das Absorptionsmittel und die daraus herstellbaren Hydrogele sollten toxikologisch und dermatologisch unbedenklich sein.

Erfindungsgemäß wird die gestellte Aufgabe gelöst durch Angabe einer vernetzten Carboxymethylstärke als Absorptionsmittel, wobei die Vernetzung teilweise oder vollständig über Dietherbrücken des Typs -O-CH(COO⁻)-O- oder -O-CH(COOH)-O- oder -O-C(CH₃)(COO⁻)-O- oder -O-C(CH₃)(COOH)-O- oder -O-CH(COCH₃)-O- erfolgt, die zwischen benachbarten Stärke-Polymerketten ausgebildet sind.

Die Erfindung beruht auf der überraschenden Erkenntnis, dass vernetzte Carboxymethylstärken, welche vernetzende Dietherbrücken umfassen, nicht nur im Unterschied zu den aus der DE 196 54 745 C2 bekannten Absorptionsmitteln mit Esterbrücken eine überraschend hohe Langzeitstabilität besitzen, sondern gleichzeitig auch die weiteren vorstehend angegebenen Erfordernisse in hervorragender Weise erfüllen.

Bei der besonders bevorzugten Verwendung von Dichloressigsäure als Vernetzungsmittel wird die Vernetzung beispielsweise teilweise oder vollständig über Dietherbrücken des Typs -O-CH(COO⁻)-O- oder -O-CH(COOH)-O- bewirkt, was besonders vorteilhaft ist. Bei der ebenfalls bevorzugten Verwendung von Dichlorpropionsäure als Vernetzungsmittel wird die Vernetzung teilweise oder vollständig über Dietherbrücken des Typs -O-C(CH₃)(COO⁻)-O- oder -O-C(CH₃)(COOH)-O- bewirkt.

Aufgrund der erfindungsgemäßen Anwesenheit der genannten Typen von Dietherbrücken besitzen die erfindungsgemäßen vernetzten Carboxymethylstärken im Vergleich mit bekannten über Etherbrücken vernetzten Carboxymethylstärken eine besonders hohe Absorptionsfähigkeit gegenüber wässrigen Flüssigkeiten. Dies gilt insbesondere für die Carboxy-funktionalisierten Dietherbrücken.

Vorteilhafterweise liegt das Vernetzerverhältnis F_{Z} (= Verhältnis Stoffmenge Vernetzungsmittel : Stoffmenge an Anhydroglucoseeinheit) im Bereich von 0,025 bis 0,5. Dies bedeutet, dass bei der Herstellung des erfindungsgemäßen Absorptionsmittels vorteilhafterweise das Vernetzungsmittel (z. B. Dichloressigsäure) in einer Konzentration von 0,025 mol - 0,5 mol pro 1 mol Stärkemonomere (Anhydroglucoseeinheit) eingesetzt wird. Bei Einhaltung des besagten Vernetzerverhältnisses ergeben sich langzeitstabile Absorptionsmittel mit einem hohen Absorptionsvermögen. Ein besonders hoher freier Quellungsgrad (Free Swelling Capacity FSC) wird erhalten, wenn das Vernetzerverhältnis F_{Z} einen Wert < 0,1 besitzt. Derart niedrige Vernetzerverhältnisse sind deshalb in vielen Fällen erwünscht.

Der Substitutionsgrad (DS) der erfindungsgemäßen vernetzten Carboxymethylstärke liegt hinsichtlich des Carboxymethylsubstituenten vorzugsweise im Bereich von 0,1 - 2 und besonders bevorzugt im Bereich von 0,2 - 1,3. Ein höherer Substitutionsgrad als 1,3 führt zu erhöhten Kosten, die nicht in jedem Falle akzeptiert werden können, insbesondere weil durch höhere Substitutionsgrade nicht sehr viel größere effektive Substitutionsgrade erreichbar sind. Ein niedrigerer Substitutionsgrad als 0,1 führt zu einer in der Regel nicht ausreichenden Absorptionskapazität.

Vorzugsweise ist eine erfindungsgemäße vernetzte Carboxymethylstärke durch Carboxymethylierung und Vernetzung nativer Stärke erhältlich. Als native Stärke können insoweit insbesondere eingesetzt werden: Kartoffelstärke (besonders bevorzugt), Maisstärke, Reisstärke oder Weizenstärke; andere native Stärken können ebenfalls eingesetzt werden.

In manchen Fällen ist es andererseits erwünscht, die erfindungsgemäße vernetzte Carboxymethylstärke nicht durch Carboxymethylierung und Vernetzung nativer Stärke zu erzeugen; in diesen Fällen wird von modifizierter und/oder derivatisierter Stärke ausgegangen.

Die Eigenschaften der erfindungsgemäßen vernetzten Carboxymethylstärke variieren insbesondere in Abhängigkeit von Substitutionsgrad (DS) und dem Vernetzerverhältnis F_{Z}. Der Fachmann wird daher den Substitutionsgrad und/oder das Vernetzerverhältnis so einstellen, wie es der Verwendungszweck der herzustellenden erfindungsgemäßen vernetzten Carboxymethylstärke erfordert.

Die erfindungsgemäßen vemetzten Carboxymethylstärken besitzen eine hohe Aufnahmekapazität und Absorptionsgeschwindigkeit für wässrige Flüssigkeiten, und zwar auch unter erhöhten Drücken. Bei Kontakt mit Wasser verkleben die äußeren Schichten der erfindungsgemäßen vernetzten Carboxymethylstärke nicht und verhindern somit auch nicht das weitere Vordringen des Wassers in das Absorptionsmittel; es besteht somit keine Neigung zum Gelblocking. Die erfindungsgemäßen vernetzten Carboxymethylstärken sind mechanisch stabil und besitzen eine hohe Gelstabilität. In gequollenem Zustand separieren die erfindungsgemäßen vernetzten Carboxymethylstärken weitgehend in einzelne Partikel.

Ein besonderer Vorteil der erfindungsgemäßen vernetzten Carboxymethylstärken liegt darin, dass sie sowohl langzeitstabil als auch aus nachwachsenden Rohstoffen herstellbar (und somit auch gesundheitlich unbedenklich sowie biologisch abbaubar) sind. Diese Eigenschaftskombination war bislang im Stand der Technik ohne Vorbild.

Die erfindungsgemäßen vemetzten Carboxymethylstärken besitzen ein ausgewogenes Verhältnis zwischen Absorptionsvermögen (charakterisiert durch das freie Quellvermögen, die Free Swelling Capacity FSC) und Gelfestigkeit. Die erfindungsgemäßen vernetzten Carboxymethylstärken ermöglichen die Aufnahme einer wässrigen Flüssigkeit auch gegen einen gleichzeitigen Druck oder gegen die Einwirkung von Scherkräften. Dies ist insbesondere dann von Bedeutung, wenn die erfindungsgemäßen vernetzten Carboxymethylstärken im Hygienebereich eingesetzt werden sollen.

Wie bereits erwähnt, betrifft die vorliegende Erfindung auch Hydrogele. Ein erfindungsgemäßes Hydrogel umfasst eine erfindungsgemäße vernetzte Carboxylmethylstärke und Wasser. Ein solches Hydrogel kann des Weiteren (a) ein oder mehrere Konservierungsmittel und/oder (b) Glycerin und/oder 1,2-Propandiol (zur Verbesserung des Hautgefühls) umfassen.

Ein erfindungsgemäßes Hydrogel kann zur Verwendung als Kontaktgeber bei medizinischen Untersuchungen eingesetzt werden. Erfindungsgemäße Hydrogele behindern Ultraschallsignale nicht oder zumindest nicht in nennenswert stärkerem Umfang als handelsübliche Ultraschallgele.

Insbesondere können die erfndungsgemäß vernetzten Carboxymethylstärken wie folgt eingesetzt werden:
1. Als Gleitgele für die Medizintechnik (z.B. Applikation von Kathetern und Sonden) und im Hygienebereich;
2. als Sonnenschutzgele für den UV-Bereich;
3. als Schutzgele mit Kühlwirkung gegen starke IR-Strahlung (z.B. für das Gesicht beim Arbeiten an Öfen);
4. als Hydratisierungsgele für trockene Haut;
5. als dermal applizierbare Gele mit Wirkstoffen wie z.B. antimikrobielle Substanzen;
6. als spezielle Dermatika für Allergiker (da die erfindungsgemäßen Hydrogele keine Emulgatoren erfordern);
7. als Quellstoff für die verzögerte Freigabe (sustained release) von Wirkstoffen in oralen Zubereitungen (z.B. Retard-Tabletten).

Die Verwendung der erfindungsgemäßen vernetzten Carboxymethylstärke ist also nicht auf den Einsatzbereich der Hydrogele beschränkt. Die Erfindung betrifft vielmehr auch Produkte zum Absorbieren, Zurückhalten oder langsamen Freisetzen einer wässrigen Flüssigkeit, d. h. von Wasser, einer wässrigen Lösung oder einer wässrigen Dispersion (insbesondere Urin oder Blut), die eine wirksame Menge einer erfindungsgemäßen vernetzten Carboxymethylstärke umfassen. Bei derartigen Produkten kann es sich um Hygieneprodukte handeln, wie z. B. (Einweg-)Windeln, Inkontinenzartikel oder Sanitärartikel zur Absorption von (wässrigen) Fluiden und zum Binden von Gerüchen. Die erfindungsgemäßen vernetzten Carboxymethylstärken können auch in Produkten eingesetzt werden, die in kontrollierter Weise langsam Wasser und gegebenenfalls darin gelöste oder dispergierte Nähr- oder Wirkstoffe an eine Umgebung abgeben; insbesondere kann es sich hierbei um pharmazeutische Produkte (controlled drug release), um Bewässerungsprodukte zur Bewässerung trockener Böden oder dergleichen handeln. Weitere erfindungsgemäße Produkte, die eine wirksame Menge einer erfindungsgemäßen vernetzten Carboxymethylstärke umfassen, sind Verpackungsmaterialien und Pflanzenkulturgefäße. Darüber hinaus können die erfindungsgemäßen vernetzten Carboxymethylstärken ganz allgemein in Produkttypen eingesetzt werden, die bislang andere Absorptionsmittel (Superabsorber) umfassen.

Die Erfindung betrifft auch ein Verfahren zur Herstellung einer erfindungsgemäßen Carboxymethylstärke; das erfindungsgemäße Verfahren umfasst dabei die folgenden Schritte:
- Bereitstellen von Stärke (native Stärke oder eine vorbehandelte oder derivatisierte Stärke),
- Vernetzen und Carboxymethylieren der Stärke zur vernetzten Carboxymethylstärke,
wobei alternativ
(a) die Stärke zunächst vernetzt und dann carboxymethyliert
   oder
(b) die Stärke zunächst carboxymethyliert und dann vernetzt
   oder
(c) die Stärke gleichzeitig vernetzt und carboxymethyliert (Eintopfverfahren)
wird.

Bevorzugt sind hierbei in vielen Fällen die Verfahrensalternativen (b) und (c). Verfahrensalternative (c) ist besonders bevorzugt, insbesondere im Vergleich mit mehrstufigen Verfahren, wie sie nach dem Stand der Technik bei Herstellung ethervernetzter Carboxymethylstärkepolymere eingesetzt werden.

Die bereitgestellte Stärke wird regelmäßig vor dem Vernetzen und Carboxylmethylieren alkalisiert und nach dem Vernetzen und Carboxymethylieren neutralisiert. Diese Verfahrensgestaltung erleichtert die Durchführung der Veretherungsreaktionen.

Gemäß einer besonders bevorzugten Ausgestaltung des erfindungsgemäßen Verfahrens wird nach dem Vemetzen und Carboxymethylieren der Stärke zur vemetzten Carboxymethylstärke die verbliebene unvernetzte Carboxymethylstärke abgetrennt. Zur Abtrennung der unvernetzten Stärke / Carboxymethylstärke von der vernetzten Carboxymethylstärke wird das nach der Carboxymethylierung und der Vernetzung vorliegende Produkt vorteilhafterweise mit Wasser (bevorzugt destilliertem oder entionisiertem Wasser) gewaschen, so dass hierbei die unvernetzten Polymeranteile (die unvernetzte Stärke / Carboxymethylstärke) aus dem Netzwerk der erfindungsgemäßen vernetzten Carboxymethylstärke herausgelöst werden. Die Waschung wird hierbei vorteilhafterweise so oft wiederholt, bis der gewünschte Reinigungserfolg erreicht ist. Der Waschvorgang kann besonders einfach über die Messung der Leitfähigkeit des Waschwassers verfolgt werden.

Zur Herstellung der erfindungsgemäßen vernetzten Carboxymethylstärken werden die Reaktionsbedingungen und/oder Reaktanten so gewählt, wie es erforderlich ist. Soll eine Dietherbrücke des Typs -O-CH(COO⁻)-O- oder -O-CH(COOH)-O- oder -O-C(CH₃)(COO⁻)-O- oder -O-C(CH₃)(COOH)-O- oder -O-CH(COCH₃)-O- hergestellt werden, wird als Reaktionspartner für die eingesetzte Stärke vorteilhafterweise Dichloressigsäure, Dichlorpropionsäure oder 1,1-Dichloraceton, oder deren Salze eingesetzt.

Vorteilhaft ist es, die Vernetzungsreaktion so zu gestalten, dass das Verhältnis von Vernetzungsmitteln (z. B. Dichloressigsäure) zu Anhydroglucoseeinheiten der eingesetzten Stärke im Bereich von F_{Z} = 0,025 bis 0,5 liegt.

Die Menge des Carboxymethylierungsmittels bei Durchführung der Carboxymethylierungsreaktion wird vorteilhafterweise so gewählt, dass ein Substitutionsgrad der vernetzten Carboxymethylstärke hinsichtlich des Carboxymethylsubstituenten im bevorzugten Bereich von 0,1 - 2 erreicht wird, vorzugsweise ein Substitutionsgrad im Bereich von 0,2 - 1,3.

Besonders bevorzugt ist ein erfindungsgemäßes Verfahren, bei dem Dichloressigsäure als Mittel zur Vernetzung der Stärke und/oder Monochloressigsäure als Mittel zur Carboxymethylierung der Stärke eingesetzt wird. Alternativ können auch die Salze der Dichloressigsäure und der Monochloressigsäure eingesetzt werden.

Als Stärke kann vorteilhafterweise wieder eine native Stärke eingesetzt werden; alternativ ist jedoch der Einsatz von modifizierter Stärke und/oder derivatisierter Stärke möglich.

Vorteilhaft ist es, die Umsetzung der Stärke zur erfindungsgemäßen vernetzten Carboxymethylstärke in wässrig-alkoholischer Suspension durchzuführen.

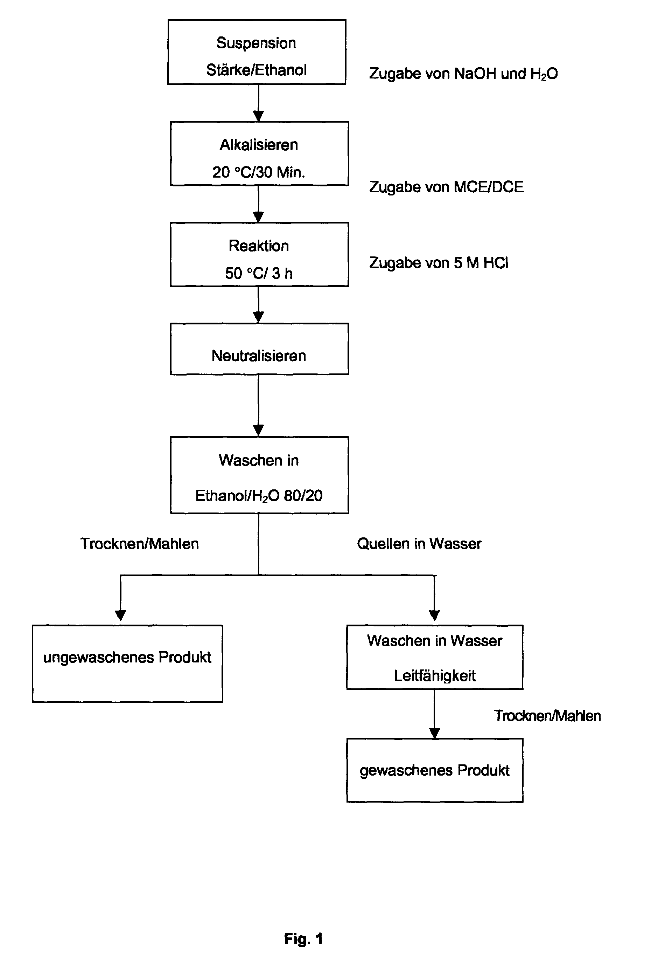

Ein typischer Verfahrensablauf zur Herstellung einer erfindungsgemäßen vernetzten Carboxymethylstärke wird in Figur 1 in Form eines Flussdiagramms anhand eines Beispiels erläutert. Es versteht sich, dass in dem Flussdiagramm jede einzelne enthaltene Angabe lediglich als Beispiel zu verstehen ist und der Fachmann von der Verfahrensgestaltung gemäß Flussdiagramm im Einzelfall abweichen kann und wird.

Es wurde bereits erwähnt, dass nach dem Vernetzen und Carboxymethylieren der Stärke zur vernetzten Carboxymethylstärke vorteilhafterweise die verbliebene unvernetzte Stärke abgetrennt wird. Vorteilhafterweise werden die unvernetzten Stärke-Polymeranteile aus der Gelmatrix entfernt, indem zunächst das Rohprodukt mit Wasser behandelt wird, wobei es aufquillt. Hierdurch werden unvernetzte Polymerstränge aus der Gelstruktur befreit und im Wasser gelöst. Anschließend kann die vernetzte Gelmatrix (die erfindungsgemäße vemetzte Carboxymethylstärke) von den unvernetzten, gelösten Polymeranteilen (der verbliebenen unvernetzten Stärke / Carboxymethylstärke) auf verschiedene Weise abgetrennt werden.

Besonders bevorzugte Verfahrensgestaltungen umfassen einen Filtrationsschritt, bei dem ein vorgequollenes Produktgemisch von eingesetztem Waschwasser (in dem die unvernetzte Stärke / Carboxymethylstärke gelöst vorliegt) abgetrennt wird. Besonders bevorzugt ist die Kontrolle der Konzentration an unvernetzten Stärkederivaten im Waschwasser mittels einer Leitfähigkeitsmessung.

In eigenen Untersuchungen haben sich folgende Maßnahmen besonders bewährt:
1. Nach einer längeren Quellzeit von z. B. 24 Stunden wird das Gemisch Gel-Polymerlösung durch Filtration über ein Filterpapier aufgetrennt. Der Vorgang (Quellen, Filtrieren) wird mehrfach wiederholt (z. B. 2-oder 3-mal).
2. Das gequollene Reaktionsprodukt (Gemisch Gel-Polymerlösung) wird in einen wasserdurchlässigen Sack aus Acetatgewebe überführt und dieser verschlossen. Der gefüllte Sack wird in ein temperiertes Wasserbad (Temperatur 40°C, destilliertes Wasser) überführt, das als Waschwasser dient. Der Waschvorgang wird mehrfach wiederholt. Die in das Waschwasser überführten unvernetzten Polymeranteile (unvernetzte Stärkederivate) lassen sich im Waschwasser beispielsweise mittels Leitfähigkeitsmessungen nachweisen; die herausgewaschenen Polymerstränge enthalten nämlich ionische Gruppen, welche die Leitfähigkeit des destillierten oder entsalzten Wassers erhöhen. Ein Durchkneten des gequollenen Reaktionsproduktes (Hydrogel) im Stoffsack beschleunigt den Waschvorgang. Vorteilhafterweise wird der Waschvorgang so oft wiederholt, bis die Leitfähigkeit des Waschwassers einen vorgegebenen Leitfähigkeitswert von etwa 30 µS/cm nicht mehr überschreitet. Dabei wird davon ausgegangen, dass die Leitfähigkeit des frischen Wassers κ_{H2O} < 10 µS/cm ist. Das Volumen des Waschwassers ist dabei geringfügig größer, als zur vollständigen Quellung des Gels benötigt wird. Das im Waschwasser enthaltene unvernetzte Stärkepolymer kann getrocknet und einem Vernetzungszyklus unterworfen werden.
3. Wie 2., der Waschvorgang erfolgt jedoch kontinuierlich in fließendem Waschwasser.
4. Das gequollene Reaktionsprodukt (Hydrogel) wird in eine Säule gefüllt, die unten mit einer Glasfritte abschließt. Ein ständiger Zustrom von destilliertem oder entsalztem Wasser wird auf die Säule gegeben. Unterhalb der Glasfritte wird das Waschwasser aufgefangen und vorteilhafterweise in regelmäßigen Abständen dessen Leitfähigkeit überprüft.
5. Das vorgequollene Reaktionsprodukt (Hydrogel) wird in einen Dialyseschlauch (vorzugsweise 14000 - 18000 Dalton) überführt und in einem Wasserbad aufgereinigt, wie unter 2. beschrieben.
6. Vorgehensweise gemäß 5., jedoch mit Aufreinigungsschritt wie unter 3. beschrieben.

Gemäß dem Flussdiagramm gemäß Figur 1 wurden insgesamt 23 Beispielprodukte hergestellt. Dabei war das Herstellungsverfahren immer im Wesentlichen das Gleiche und genügt der nachfolgenden allgemeinen Verfahrensvorschrift.

Allgemeine Verfahrensvorschrift:
Zunächst wird in einem Temperierbecher (20°C) eine Kartoffelstärke-Alkohol-Suspension vorgelegt, die durch Zugabe einer definierten Menge NaOH und Wasser unter kräftigem Rühren für 30 Minuten alkalisiert wird. Dazu werden gleichzeitig die vorab berechneten Mengen Monochloressigsäure und Dichloressigsäure gegeben und das Reaktionsgemisch unter Rühren auf die gewünschte Reaktionstemperatur erwärmt. Nach 3stündiger Reaktionszeit wird das Produkt abgesaugt und in ein Ethanol-Wasser-Gemisch (80 % Alkohol) überführt. Anschließend wird mittels wässriger HCl-Lösung (5N) der pH-Wert zum neutralen Bereich eingestellt. Die durch die Neutralisation erzeugte Salzfracht (NaCl) wird durch 3maliges Auswaschen in Alkohol/Wasser (80%/20%) reduziert. Danach wird das Produkt in reines Wasser überführt und nach einer Quellzeit von 24 h den Waschvorgängen zugeführt, bis die unvernetzten Polymeranteile und die Salzfracht nahezu quantitativ entfernt wurden. Das gereinigte Produkt wird getrocknet und vermahlen. Das Hydrogel kann dann in gewünschter Konzentration mit reinem Wasser angesetzt werden, wobei auch Konservierungsstoffe zur längeren Haltbarkeit zugesetzt werden können.

Für alle Beispiele 1 - 23 gilt:
MCE = Monochloressigsäure oder deren Salze = Carboxymethylierungsmittel
DCE = Dichloressigsäure oder deren Salze = Vernetzer
AGE = Anhydroglucoseeinheit (Stärkemonomer)
Gesamtmasse des Syntheseansatzes (Masse der Reaktanden + Lösemittel + Wasser) = 500 g
Alkalisierungsverhältnis n_{NaOH}/n_{AGE} = 3
Vernetzerverhältnis F_{Z} = n_{DCE}/n_{AGE} = 0,1 (abweichende Werte werden in den Beispielen 1 - 23 aufgeführt)
Substituentenverhältnis s= n_{MCE}/n_{AGE} = 1 (abweichende Werte werden in den Beispielen 1 - 23 aufgeführt)
Wasseranteil in Massen%/Gesamtmasse = 15 (abweichende Werte werden in den Beispielen 1 - 23 aufgeführt)
Lösemittel = Ethanol (andere Lösungsmittel werden in den Beispielen 1 - 23 genannt)
Temperatur T = 50°C (abweichende Werte werden in den Beispielen 1 - 23 aufgeführt)

**Beispiel 1:**
Produkt hergestellt aus 50 g Kartoffelstärke, 29, 17 g MCE, 2,01 g DCE, F_{Z} = 0,05

**Beispiel 2:**
Produkt hergestellt aus 50 g Kartoffelstärke, 29, 17 g MCE, 4,02 g DCE

**Beispiel 3:**
Produkt hergestellt aus 50 g Kartoffelstärke, 29, 17 g MCE, 8,04 g DCE, F_{Z} = 0,2

**Beispiel 4:**
Produkt hergestellt aus 50 g Kartoffelstärke, 29, 17 g MCE, 20, 11 g DCE, F_{Z} = 0,5

**Beispiel 5:**
Produkt hergestellt aus 50 g Kartoffelstärke, 29, 17 g MCE, 1,01 g DCE, F_{Z} = 0,025

**Beispiel 6:**
Produkt hergestellt aus 50 g Kartoffelstärke, 29,17 g MCE, 4,02 g DCE, T = 78,7°C

**Beispiel 7:**
Produkt hergestellt aus 62,50 g Kartoffelstärke, 44,95 g MCE (Na-Salz), 5,03 g DCE, Stärkeanteil 12,5 Massen%

**Beispiel 8:**
Produkt hergestellt aus 75 g Kartoffelstärke, 53,94 g MCE (Na-Salz), 6,04 g DCE, Stärkeanteil 15 Massen%

**Beispiel 9:**
Produkt hergestellt aus 87,5 g Kartoffelstärke, 62,93 g MCE (Na-Salz), 7,04 g DCE, Stärkeanteil 17,5 Massen%

**Beispiel 10:**
Produkt hergestellt aus 100 g Kartoffelstärke, 71,92 g MCE (Na-Salz), 8,05 g DCE, Stärkeanteil 20 Massen%

**Beispiel 11:**
Produkt hergestellt aus 50 g Kartoffelstärke, 35,96 g MCE (Na-Salz), 4,02 g DCE, Lösemittel Methanol

**Beispiel 12:**
Produkt hergestellt aus 75 g Kartoffelstärke, 53,94 g MCE (Na-Salz), 6,04 g DCE, Lösemittel Methanol, Stärkeanteil 15 Massen%

**Beispiel 13:**
Produkt hergestellt aus 100 g Kartoffelstärke, 71,92 g MCE (Na-Salz), 8,05 g DCE, Lösemittel Methanol, Stärkeanteil 20 Massen%

**Beispiel 14:**
Produkt hergestellt aus 50 g Kartoffelstärke, 35,96 g MCE (Na-Salz), 4,02 g DCE, Lösemittel Isopropanol (Propan-2-ol), s = 1

**Beispiel 15:**
Produkt hergestellt aus 50 g Kartoffelstärke, 53,94 g MCE (Na-Salz), 4,02 g DCE, Lösemittel Isopropanol (Propan-2-ol), s = 1,5

**Beispiel 16:**
Produkt hergestellt aus 50 g Kartoffelstärke, 71,92 g MCE (Na-Salz), 4,02 g DCE, Lösemittel Isopropanol (Propan-2-ol), s = 2

**Beispiel 17:**
Produkt hergestellt aus 50 g Kartoffelstärke, 89,89 g MCE (Na-Salz), 4,02 g DCE, Lösemittel Isopropanol, s = 2,5

**Beispiel 18:**
Produkt hergestellt aus 50 g Kartoffelstärke, 107,87 g MCE (Na-Salz), 4,02 g DCE, Lösemittel Isopropanol, s = 3

**Beispiel 19:**
Produkt hergestellt aus 50 g Wachsmaisstärke, 29,17 g MCE, 4,02 g DCE

**Beispiel 20:**
Produkt hergestellt aus 50 g Wachsmaisstärke, 29,17 g MCE, 4,02 g DCE, Wasseranteil = 12 %

**Beispiel 21:**
Produkt hergestellt aus 50 g Wachsmaisstärke, 29,17 g MCE, 4,02 g DCE, Wasseranteil = 10 %

**Beispiel 22:**
Produkt hergestellt aus 50 g Amylomaisstärke, 35,96 g MCE (Na-Salz), 4,02 g DCE, Wasseranteil = 10 %

**Beispiel 23:**
Produkt hergestellt aus 50 g Amylomaisstärke, 35,96 g MCE (Na-Salz), 4,02 g DCE, Wasseranteil 14 %

In der nachfolgenden Tabelle 1 sind wesentliche Daten der Herstellungsverfahren gemäß den Beispielen 1 - 23 zusammengefasst. Zudem enthält Tabelle 1 Angaben zum Vernetzerverhältnis n_{DCE}/n_{AGE} und zum ermittelten Substitutionsgrad. Das Vernetzerverhältnis bezieht sich dabei auf die Edukte.

Zu den Bedeutungen der in Tabelle 1 enthaltenen Spalten und den in Tabelle 1 verwendeten Abkürzungen siehe die folgenden Erläuterungen:

### Erläuterungen:

Spalte 1: Beispiel-Nummer
Spalte 2: Stärkeart
Spalte 3: Einwaage der Stärke in Gramm
Spalte 4: Massenanteil der Stärke des Gesamtansatzes
Spalte 5: Einwaage Monochloressigsäure in Gramm
Spalte 6: Substituentenverhältnis n_{MCE}/n_{AGE}
Spalte 7: Einwaage Dichloressigsäure in Gramm
Spalte 8: Vernetzerverhältnis F_{Z} = n_{DCE}/n_{AGE}
Spalte 9: Reaktionstemperatur in °C
Spalte 10: Verwendetes Lösemittel
Spalte 11: Massenanteil des Wassers im Gesamtansatz
Spalte 12: Ermittelter Substitutionsgrad (DS)
Spalte 13: Ermittelter freier Quellungsgrad (FSC)

Abkürzungen:
KS = Kartoffelstärke
AM = Amylomaisstärke
WM = Wachsmaisstärke
MCE = Monochloressigsäure
MCE/Na = Monochloressigsäure-Natrium-Salz
DCE = Dichloressigsäure
S = Substituentenverhältnis
F_{Z} = Vernetzerverhältnis
T = Reaktionstemperatur
DS = Degree of Substitution (Substitutionsgrad)
FSC = Free Swelling Capacity (Freier Quellungsgrad)

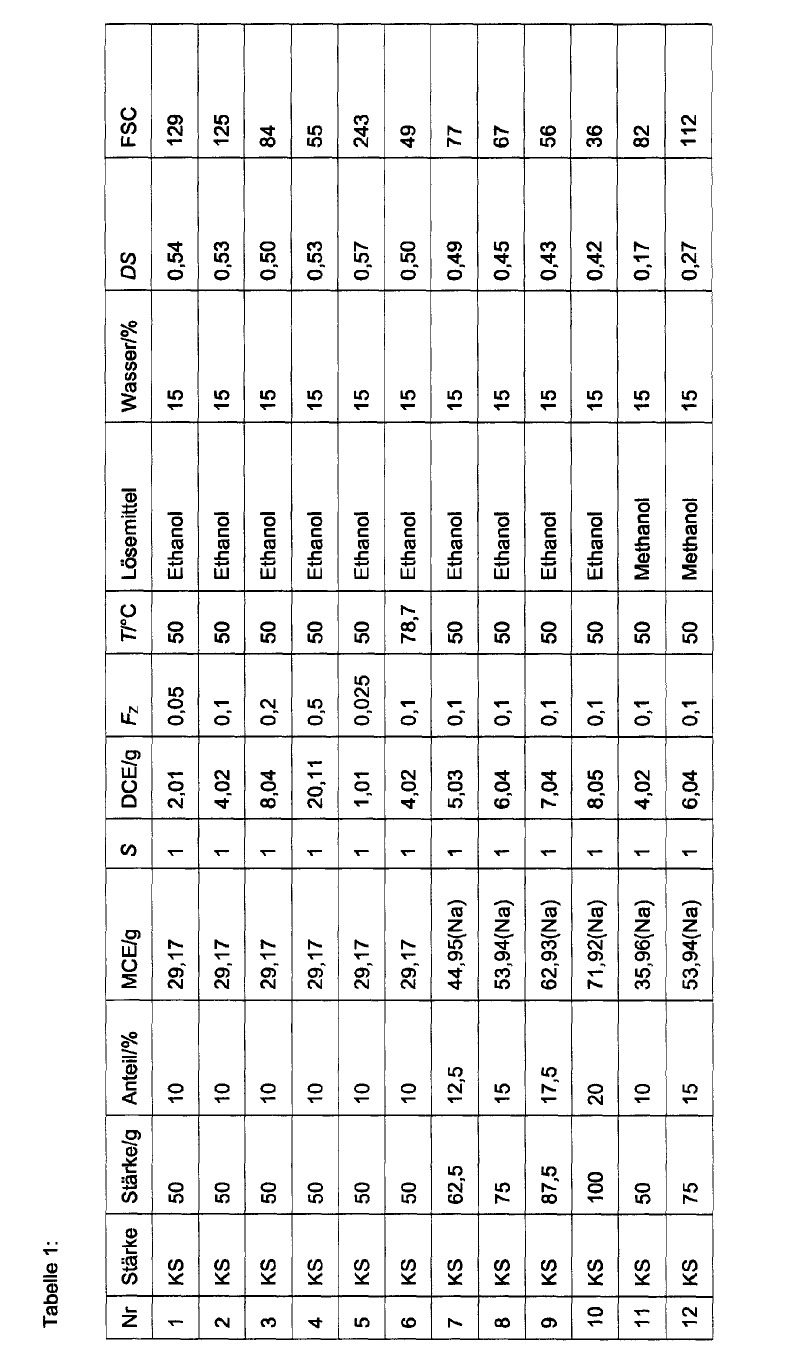

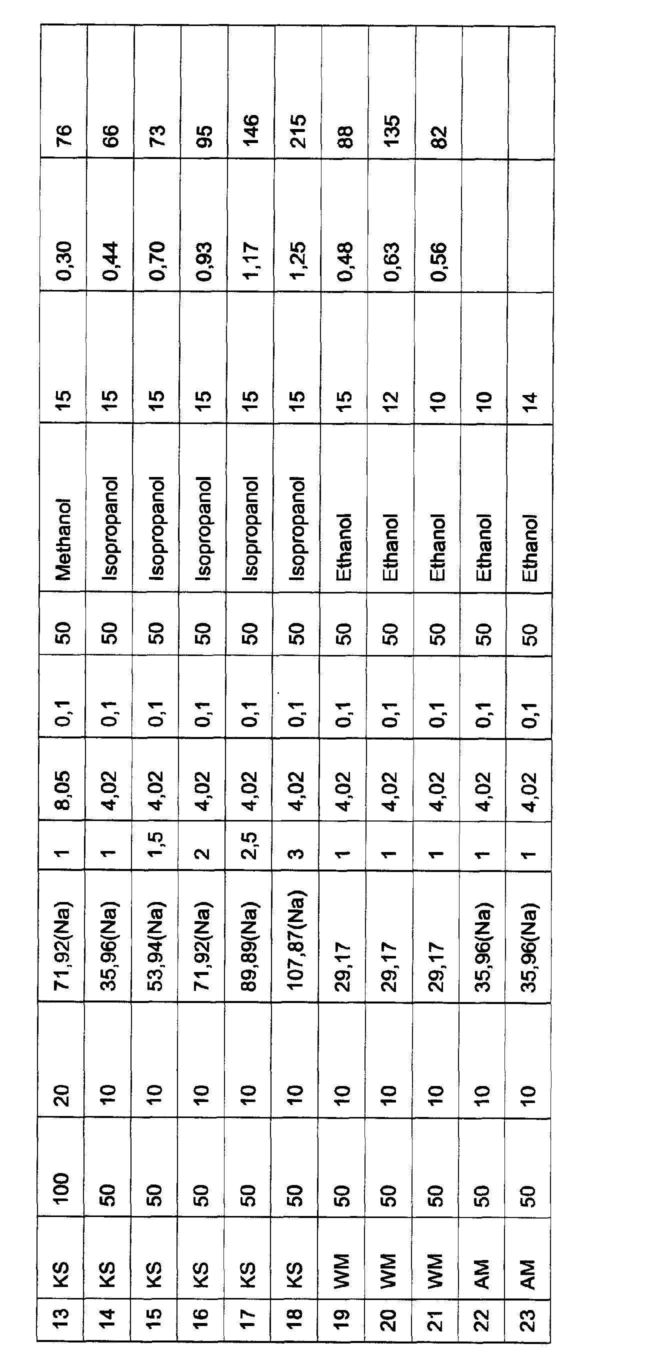

## Patentansprüche

1. Vernetzte Carboxymethylstärke zur Verwendung als Absorptionsmittel für eine wässrige Flüssigkeit, wobei die Vernetzung teilweise oder vollständig über Dietherbrücken des Typs -O-CH(COO⁻)-O- oder -O-CH(COOH)-O- oder -O-C(CH₃)(COO⁻)-O- oder -O-C(CH₃)(COOH)-O- oder -O-CH(COCH₃)-O- erfolgt, die zwischen benachbarten Stärke-Polymerketten ausgebildet sind.

2. Vernetzte Carboxymethylstärke nach einem der vorangehenden Ansprüche, wobei das Vernetzerverhältnis F_{Z} im Bereich von 0,025 bis 0,5 liegt.

3. Vernetzte Carboxymethylstärke nach einem der vorangehenden Ansprüche, wobei der Substitutionsgrad der vernetzten Carboxymethylstärke hinsichtlich des Carboxymethylsubstituenten im Bereich von 0,1 bis 2 liegt, vorzugsweise im Bereich von 0,2 bis 1,3.

4. Vernetzte Carboxymethylstärke nach einem der vorangehenden Ansprüche, erhältlich durch Carboxymethylierung und Vernetzung nativer Stärke.

5. Hydrogel umfassend
- eine vernetzte Carboxymethylstärke nach einem der vorangehenden Ansprüche und
- Wasser.

6. Hydrogel nach Anspruch 5, weiter umfassend (a) ein oder mehrere Konservierungsmittel und/oder (b) Glycerin und/oder 1,2-Propandiol.

7. Produkt zum Absorbieren, Zurückhalten oder langsamen Freisetzen einer wässrigen Flüssigkeit, umfassend eine wirksame Menge einer vernetzten Carboxymethylstärke nach einem der Ansprüche 1-4.

8. Verfahren zur Herstellung einer vernetzten Carboxymethylstärke nach einem der Ansprüche 1-4, mit folgenden Schritten:
- Bereitstellen von Stärke,
- Vernetzen und Carboxymethylieren der Stärke zur vernetzten Carboxymethylstärke,
wobei alternativ
(a) die Stärke zunächst vernetzt und dann carboxymethyliert
oder
(b) die Stärke zunächst carboxymethyliert und dann vernetzt
oder
(c) die Stärke gleichzeitig vernetzt und carboxymethyliert
wird.

9. Verfahren nach Anspruch 8, wobei die bereitgestellte Stärke vor dem Vernetzen und Carboxymethylieren alkalisiert und nach dem Vernetzen und Carboxymethylieren neutralisiert wird.

10. Verfahren nach Anspruch 8 oder 9, wobei nach dem Vernetzen und Carboxymethylieren der Stärke zur vernetzten Carboxymethylstärke verbliebene unvernetzte Carboxymethylstärke abgetrennt wird.

11. Verfahren nach einem der Ansprüche 8-10, wobei
- Dichloressigsäure und/oder deren Salze und/oder Dichlorpropio n-säure und/oder deren Salze als Mittel zur Vernetzung der Stärke
und/oder
- Monochloressigsäure als Mittel zur Carboxymethylierung der Stärke eingesetzt wird.

12. Verfahren nach einem der Ansprüche 8-11, wobei als Stärke native Stärke eingesetzt wird.

13. Verwendung einer vernetzten Carboxymethylstärke nach einem der Ansprüche 1-4 als Absorptionsmittel für Wasser, eine wässrige Lösung oder eine wässrige Dispersion.

14. Verwendung eines Hydrogels nach einem der Patentansprüche 5-6 (a) als Kontaktgeber bei medizinischen Untersuchungen, (b) als Gleitgel für die Medizintechnik, (c) als Sonnenschutzgel, (d) als Schutzgel mit Kühlwirkung, (e) als Hydratisierungsgel für trockene Haut, (f) als dermal applizierbares Gel in Kombination mit Wirkstoffen, (g) als emulgatorfreies Dermatikum oder (h) Quellstoff zur verzögerten Freigabe eines Wirkstoffs in einer oralen Zubereitung.
